(19) Europäisches Patentamt
European Patent Office
Office européen des brevets

(11) **EP 2 756 847 A1**

(12) **EUROPEAN PATENT APPLICATION**
published in accordance with Art. 153(4) EPC

(43) Date of publication:
**23.07.2014 Bulletin 2014/30**

(21) Application number: **12831816.9**

(22) Date of filing: **07.09.2012**

(51) Int Cl.:
*A61K 35/12* (2006.01)    *A61K 35/55* (2006.01)
*A61P 25/28* (2006.01)

(86) International application number:
**PCT/JP2012/072909**

(87) International publication number:
**WO 2013/039000 (21.03.2013 Gazette 2013/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.09.2011 JP 2011199772**

(71) Applicant: **Ochiya, Takahiro**
**Tokyo 104-0045 (JP)**

(72) Inventor: **Ochiya, Takahiro**
**Tokyo 104-0045 (JP)**

(74) Representative: **Woods, Geoffrey Corlett**
**J A Kemp**
**14 South Square**
**Gray's Inn**
**London WC1R 5JJ (GB)**

(54) **PHARMACEUTICAL PRODUCT FOR PREVENTING OR TREATING ALZHEIMER'S DISEASE**

(57)    The present invention provides a therapeutic agent for Alzheimer's disease. The therapeutic agent contains membrane vesicles (exosomes) of adipose tissue-derived mesenchymal stem cells, and the membrane vesicles (exosomes) contain neprilysin. Through the research by the inventors of the present invention, it was revealed that exosomes secreted by human adipose tissue-derived mesenchymal stem cells contain neprilysin, which degrades amyloid-β as a pathogenic protein of Alzheimer's disease. When exosomes secreted by human adipose tissue-derived mesenchymal stem cells were administered to the brains of Alzheimer's disease model mice, the generation of amyloid-β was inhibited.

Neprilysin was detected in exosomal fraction of hAT-MSC-derived culture supernatant.

FIG. 1

**Description**

Technical Field

The present invention relates to a pharmaceutical product for preventing or treating Alzheimer's disease.

Background Art

Alzheimer's disease is the most common senile dementia across the world, and is characterized by progressive cognitive impairments (such as memory problems, disorientation, learning disability, and space perception impairment). In a person with Alzheimer's disease, amyloid plaques are seen in the cerebral cortex, and amyloid-$\beta$ is the main component of the amyloid plaques. Various therapeutic agents for Alzheimer's disease have been proposed (for example, Patent Document 1). Unfortunately, however, no effective therapeutic agent is currently present.

Citation List

Patent Document(s)

Patent Document 1: JP 2011-126847 A

Summary of the Invention

Problem to be Solved by the Invention

With the foregoing in mind, it is an object of the present invention to provide a pharmaceutical product effective for preventing or treating Alzheimer's disease, and a method for preventing or treating Alzheimer's disease.

Means for Solving Problem

The present invention provides a pharmaceutical product for preventing or treating Alzheimer's disease, containing: a membrane vesicle of an adipose tissue-derived mesenchymal stem cell.
The present invention also provides a method for preventing or treating Alzheimer's disease, including: administering a membrane vesicle of adipose tissue-derived mesenchymal stem cell to an Alzheimer's disease patient.
The membrane vesicle contains neprilysin.

Effects of the Invention

The inventors of the present invention found out that neprilysin, which degrades amyloid-$\beta$, is present in membrane vesicles of adipose tissue-derived mesenchymal stem cells, and also found out, by administering the membrane vesicles, amyloid-$\beta$ is degraded so that Alzheimer's disease can be prevented or treated. Based on these findings, the inventors of the present invention achieved the present invention. According to the present invention, it is possible to provide a pharmaceutical product effective for preventing or treating Alzheimer's disease and a method for preventing or treating Alzheimer's disease.

Brief Description of Drawings

[FIG. 1] FIG. 1 shows electrophoretograms indicating the presence of neprilysin in membrane vesicles (exosomes) of adipose tissue-derived mesenchymal stem cells.
[FIG. 2] FIG. 2 shows graphs showing the activity of neprilysin in membrane vesicles (exosomes) of adipose tissue-derived mesenchymal stem cells.
[FIG. 3] FIG. 3 shows photographs of tissues, demonstrating that amyloid-$\beta$ disappeared in mice to which membrane vesicles (exosomes) of adipose tissue-derived mesenchymal stem cells had been administered. Mode for Carrying out the Invention

In the present invention, the adipose tissue-derived mesenchymal stem cells are not particularly limited, and may be derived from an Alzheimer's disease patient or a humans who is not an Alzheimer's disease patient, for example.

For example, the pharmaceutical product according to the present invention may be prepared using adipose tissue-derived mesenchymal stem cells collected from an Alzheimer's disease patient to be treated. Alternatively, the pharmaceutical product according to the present invention for an Alzheimer's disease patient to be treated may be prepared using adipose tissue-derived mesenchymal stem cells collected from any other person.

In the present invention, the membrane vesicle is a membranous substance secreted by an adipose tissue-derived mesenchymal stem cell, and the size thereof is, for example, 5 to 200 nm, preferably 10 to 100nm in diameter. The membrane vesicle is an exosome, for example. The exosome refers to, among various vesicles present in an intracellular membrane, a nanometer-order vesicle secreted to the outside of the cell, for example.

In the present invention, membrane vesicles containing neprilysin can be produced by culturing adipose tissue-derived mesenchymal stem cells, for example. A method for collecting the membrane vesicles from the cultured adipose tissue-derived mesenchymal stem cells is not particularly limited. For example, membrane vesicles released to the outside of the cells may be collected by collecting the culture supernatant, and membrane vesicles present inside the cells may be collected after disrupting the cells.

Collection of the membrane vesicles can be achieved by, for example, filtration, centrifugation, or the like. The membrane vesicles can be fractionated on the basis of their diameters, for example. As a specific example, the exosomes can be fractionated on the basis of the diameter in the range from 30 to 100 nm, for example. Specific examples of the collection method include a method in which a culture solution is centrifuged (for example, $500 \times g$, 10 minutes) to collect the supernatant, the supernatant is filtered (for example, using a 0. 22-$\mu$m filter) and then ultracentrifuged (for example, $100,000 \times g$ to $120,000 \times g$, 70 minutes), and the precipitated fraction is collected as membrane vesicles. Also, the membrane vesicles may be collected based on a density gradient using carriers, such as sucrose, with different specific gravities and then performing density gradient centrifugation. The collection of the membrane vesicles also can be carried out with reference to literatures such as Nat. Cell. Biol., 2007 Jun, 9(6), pp. 654-659; Epub 2007 May 7., PMID: 17486113; and the like.

In the present invention, a method for administering the membrane vesicles is not particularly limited, and examples thereof include administration by injection, transdermal administration, and oral administration. The administration by injection may be, for example, subcutaneous injection, intravenous injection, or the like. The dose is determined as appropriate depending on the body size, age, sex, progress of the disease, etc. of the patient.

Examples

Next, examples of the present invention will be described. It is to be noted, however, that the present invention is by no means restricted or limited by the following examples.

(Confirmation of the presence of neprilysin)

The presence of neprilysin in membrane vesicles of adipose tissue-derived mesenchymal stem cells was confirmed by the following method.

Human adipose tissue-derived mesenchymal stem cells ($1 \times 10^6$/20 ml) were cultured in a serum-free medium for 72 hours, after which the culture supernatant was collected. This culture supernatant was centrifuged at $2,000 \times g$ for 10 minutes, and cell debris and the like were removed. Thereafter, the culture supernatant further was ultracentrifuged at $100,000 \times g$ (4°C) for 70 minutes. The supernatant was then discarded, and physiological saline was added to the residue to cause precipitation. The thus-obtained precipitated fraction was used as an exosome fraction. This exosome fraction was dissolved so that the total amount of the resultant mixture was 200 ml, and the exosome fraction was purified by ultracentrifugation. The protein amount was quantified by the Bradford protein assay, and a 10 mg protein equivalent per well was used in the Western blotting. After performing PAGE using Mini-PROTEAN TGX gels of 4% to 15% (Bio-Rad), the separated proteins were transferred on PVDF membranes and subjected to blocking at 4°C overnight (Nacalai blocking solution). Next, a primary antibody reaction was carried out for 1 hour at room temperature (mouse monoclonal anti-CD10, ab951, 1 : 1000). Subsequently, a secondary antibody reaction was carried out for 1 hour at room temperature (sheep anti-mouse Ig HRP conjugated, 1 : 5000). At the end, a color-developing reaction was carried out using an Immunostar (Wako) (for 5 minutes at room temperature), and the blotting images were photographed.

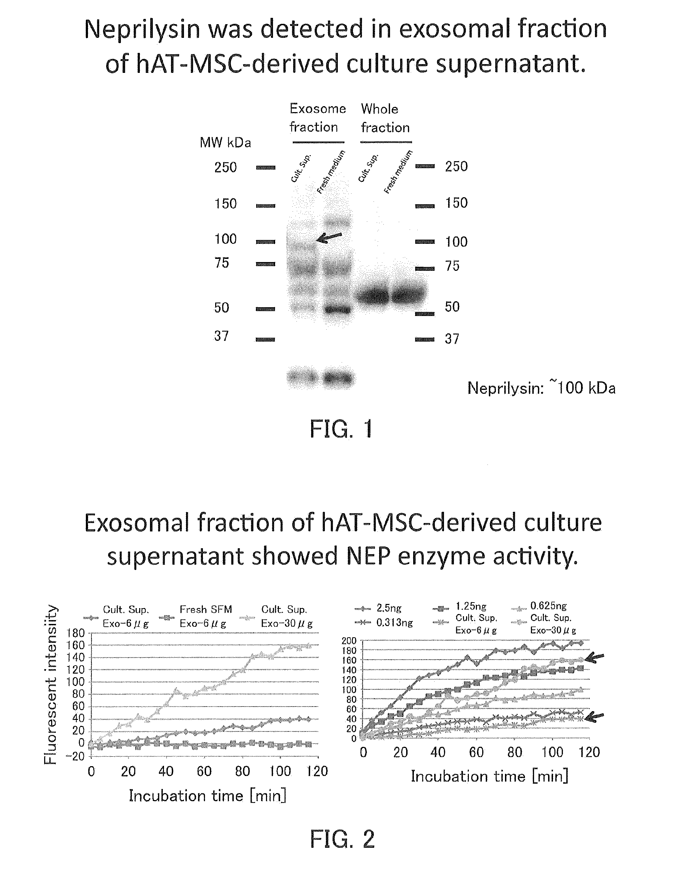

The photograph in FIG. 1 shows the result of the above-described blotting. In the photograph in FIG. 1, the band indicated with the arrow corresponds to neprilysin. This result confirmed that neprilysin is present in membrane vesicles (exosomes) of human adipose tissue-derived mesenchymal stem cells.

(Confirmation of activity of neprilysin)

Next, the activity of the neprilysin in the membrane vesicles (exosomes) obtained in the above-described manner was confirmed by the following method.

The above-described exosome fraction was dissolved in an assay buffer (100 mM Tris, 50 mM NaCl, 10 mg/mL ZnCl$_2$, pH = 7.5) to prepare a sample. Also, as standard samples, solutions containing recombinant human Neprilysin (R&D) at the respective concentrations in the range from 0.31 ng to 10 ng were used. Each sample was supplied to a 96-well black plate on ice (6 μg/well and 30 μg/well). Subsequently, an assay buffer containing a 20 mM substrate and 10mg/mL thiorphan (±) was added so that the respective wells contained equal amounts (50 mL) of the assay buffer, and then the measurement was started. During the measurement, the fluorescence intensity was measured every 5 minutes while incubating the plate at 37°C, and the kinetics of the enzyme reaction was observed.

The neprilysin activity was measured using a fluorogenic substrate Mca-RPPGFSAFK (Dnp). This is not only a substrate for neprilysin but also a substrate for several kinds of other enzymes. Thus, in the measurement of the neprilysin activity, a system containing thiorphan, which is an inhibitor specific to neprilysin, also was provided, and the activity of the enzymes other than neprilysin also was measured. The activity of the neprilysin was evaluated according to the following equation.

$$\text{Neprilysin activity} = \text{fluorescence intensity (in the absence of inhibitor)} -$$
$$\text{fluorescence intensity (in the presence of inhibitor)}$$

The graphs in FIG. 2 show the results of the neprilysin activity measurement. The neprilysin activity measurement was carried out through observation for 120 minutes after the addition of the fluorogenic substrate. When the exosomes derived from the human adipose tissue-derived mesenchymal stem cells (hAT-MSC) were added to the solution containing the fluorogenic substrate and the resultant mixture was incubated at 37°C, an increase in fluorescence intensity over time was observed, from which it was confirmed that the exosomes have enzyme activity. In contrast, in the system containing the inhibitor specific to neprilysin (NEP), the increase in fluorescence intensity decreased considerably. This revealed that the exosomes derived from the human adipose tissue-derived mesenchymal stem cells (hAT-MSC) have neprilysin (NEP) activity. Also, from the comparison with the dilution series of the recombinant human neprilysin (rhNEP), it was found that the activity of the neprilysin (NEP) derived from these exosomes was approximately equivalent to the activity of 1.25 ng of rhNEP.

(Inhibition of amyloid-β generation in membrane vesicle-administered mice)

According to the following method, membrane vesicles of human adipose tissue-derived mesenchymal stem cells were administered to Alzheimer's disease model mice, and inhibition of amyloid-β generation was confirmed.

The exosome fraction prepared in the above-described manner was dissolved so that the total amount of the resultant mixture was 200 ml. This mixture was administered to the tail veins of mice (Alzheimer's disease model mice, one month old) immediately after the preparation. As a control, 200 ml of physiological saline was administered intravenously to littermate Alzheimer's disease model mice. The administration was performed 3 times in total at intervals of 7 days. Then, 7 days after the last administration, the animals were euthanized, and their brains were harvested and fixed. Then, tissue slices centered on the hippocampus, the cerebrum, and the thalamus were prepared, and amyloid-β plaques were stained with anti-amyloid-β antibody. Some of the collected and administered exosomes were subjected to protein extraction and the Western analysis, and the presence of a CD63 antigen as an exosome marker therein was confirmed.

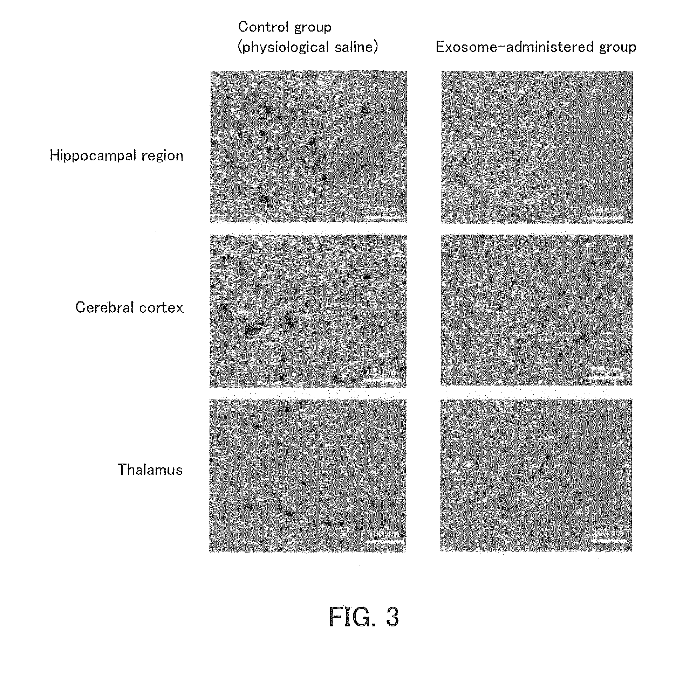

The photographs in FIG. 3 show inhibition of the amyloid-β generation by the administration of the membrane vesicles. From FIG. 3, it can be seen that the generation of amyloid-β was inhibited in the brain of the model mouse to which the membrane vesicles had been administered.

**Claims**

1. A pharmaceutical product for preventing or treating Alzheimer's disease, the pharmaceutical product comprising:

   a membrane vesicle of an adipose tissue-derived mesenchymal stem cell.

2. The pharmaceutical product according to claim 1, wherein the membrane vesicle comprises neprilysin.

3. A method for preventing or treating Alzheimer's disease, the method comprising:

administering a membrane vesicle of adipose tissue-derived mesenchymal stem cell to an Alzheimer's disease patient.

4. The method according claim 3, where the membrane vesicle comprises neprilysin.

# Neprilysin was detected in exosomal fraction of hAT-MSC-derived culture supernatant.

FIG. 1

# Exosomal fraction of hAT-MSC-derived culture supernatant showed NEP enzyme activity.

FIG. 2

Control group
(physiological saline)    Exosome-administered group

Hippocampal region

Cerebral cortex

Thalamus

FIG. 3

## INTERNATIONAL SEARCH REPORT

| International application No. |
| --- |
| PCT/JP2012/072909 |

A. CLASSIFICATION OF SUBJECT MATTER
*A61K35/12*(2006.01)i, *A61K35/55*(2006.01)i, *A61P25/28*(2006.01)i

According to International Patent Classification (IPC) or to both national classification and IPC

B. FIELDS SEARCHED

Minimum documentation searched (classification system followed by classification symbols)
A61K35/12, A61K35/55, A61P25/28

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
JSTPlus/JMEDPlus/JST7580(JDreamII), BIOSIS(STN), CAplus(STN), EMBASE(STN), MEDLINE(STN)

C. DOCUMENTS CONSIDERED TO BE RELEVANT

| Category* | Citation of document, with indication, where appropriate, of the relevant passages | Relevant to claim No. |
| --- | --- | --- |
| X | CN 101845416 A (YUAN L), 29 September 2010 (29.09.2010), entire text (Family: none) | 1,2 |
| X | NIU,Y. et al, Adipose derived mesenchymal stem cell transplantation for Alzheimer's disease, Zhongguo Zuzhi Gongcheng Yanjiu Yu Linchuang Kangfu (Journal of Clinical Rehabilitative Tissue Engineering Research), 2009, Vol.13, No. 27, p.5389-5392, particularly, Abstracts | 1,2 |
| X | WO 2003/085099 A2 (MEDESTEA INT SRL), 16 October 2003 (16.10.2003), claims; page 5 & US 2006/0110825 A1 & EP 1495114 A | 1,2 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 24 September, 2012 (24.09.12) | 02 October, 2012 (02.10.12) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**INTERNATIONAL SEARCH REPORT**

| International application No. |
|---|
| PCT/JP2012/072909 |

C (Continuation). DOCUMENTS CONSIDERED TO BE RELEVANT

| Category* | Citation of document, with indication, where appropriate, of the relevant passages | Relevant to claim No. |
|---|---|---|
| X | JP 2009-001509 A (Nagoya University), 08 January 2009 (08.01.2009), claims; paragraph [0015] (Family: none) | 1,2 |
| X | WO 2010/056075 A2 (MEDIPOST CO., LTD.), 20 May 2010 (20.05.2010), claims & JP 2012-508733 A & EP 2365816 A & KR 10-2010-0054711 A & KR 10-2010-0054731 A | 1,2 |
| P,X | JP 2012-157263 A (Seems, Inc.), 23 August 2012 (23.08.2012), claims 9 to 15; paragraphs [0038], [0039] (Family: none) | 1,2 |

Form PCT/ISA/210 (continuation of second sheet) (July 2009)

**INTERNATIONAL SEARCH REPORT**

International application No.

PCT/JP2012/072909

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 3,4
   because they relate to subject matter not required to be searched by this Authority, namely:
   (See extra sheet)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2012/072909

Continuation of Box No.II-1 of continuation of first sheet(2)

The inventions described in claims 3 and 4 relate to a method for preventing or treating Alzheimer's disease, and therefore pertain to a method for treatment by therapy. Thus, the inventions of these claims relate to a subject matter which this international searching authority is not required, under the provisions of PCT Article 17(2)(a)(i) and PCT Rule 39.1(iv), to search.

Form PCT/ISA/210 (extra sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011126847 A

**Non-patent literature cited in the description**

- *Nat. Cell. Biol.,* 07 May 2007, vol. 9 (6), 654-659